# EUROPEAN PATENT SPECIFICATION

(11) **EP 1 435 394 B1**
(45) Date of publication and mention of the grant of the patent: **27.01.2010**
(21) Application number: 03028474.9
(22) Date of filing: 12.12.2003
(51) Int. Cl.: C12Q 1/68

(54) **A method for quantifying a target nucleic acid**
Verfahren zur Quantifizierung von Nukleinsäuren
Méthode pour mesurer un acide nucléique

(30) Priority: 12.12.2002 JP 2002360914
(43) Date of publication of application: 07.07.2004
(73) Proprietor: FUJIFILM Corporation, Minato-ku Tokyo (JP)
(72) Inventor: Iwaki, Yoshihide, Asaka-shi, Saitama 351-8585 (JP); Makino, Yoshihiko, Asaka-shi, Saitama 351-8585 (JP)
(74) Representative: Hiebl, Inge Elisabeth

(56) References cited:
- EP-A- 0 333 114
- EP-A- 0 629 705
- EP-A- 0 630 974
- WO-A-02/18616
- WO-A-92/16654
- WO-A-98/55653
- GB-A- 2 324 865

## Description

### Technical Field

The present invention relates to a method for quantifying a target nucleic acid in a sample by the polymerase chain reaction.

### Background Art

Quantitative analysis of nucleic acids has become to play a more important role in the fields of biology and medicine. Regarding cancerous genes in cancerous tissues, for example, a gene is quantitatively analyzed in order to determine the level of specific gene.

Also, quantification of HCV-RNA or HIV-RNA is utilized in diagnosis or prediction or judgment of therapeutic effects.

Particularly, the quantitative assay of HCV-RNA has been put into practice for the purpose of therapy. Many patients with chronic active hepatitis C take IFN therapy, and the factor that determines the effect of IFN therapy is considered to be the quantitative difference of HCV (Naoya Kato et al., Kanzo (Liver), 1991; 32; 750-751). Thus, the effect of IFN therapy can be directly found by monitoring the amount of virus during IFN therapy. This enables more effective IFN therapy that is tailored to clinical conditions of each patient.

Further, quantification of the target nucleic acid is considered to provide information which is also important for diagnosis of diseases in the future. For example, earlier diagnosis can be effected by examining the expression level of mRNA that responds to exogenous stimuli in the case of a disease that results from exogenous stimuli.

The polymerase chain reaction (PCR, U.S. Patent Nos. 4,683,195 and 4,683,202) that is a technique of nucleic acid amplification, is excellent as a technique of highly sensitive analysis for detecting a trace amount of nucleic acids. This technique enabled the detection of a trace amount of mRNA, which was conventionally difficult to be detect, and the detection of mRNA in a small amount of tissue or cell.

When PCR is employed, however, the absolute amount of the amplified nucleic acids does not accurately reflect the amount of the target nucleic acid that had existed when amplification was initiated.

At first, the amount of the product amplified by PCR exponentially increases every cycle, however, the rate of increase slows down when the amount of the amplified product exceeds a certain level, and the amplified product finally stops increasing. Thus, the final amount of the amplified product is constant regardless of the amount of the target nucleic acid when the reaction was initiated. This phenomenon is referred to as the plateau effect, which should be taken into consideration when quantifying the product amplified by PCR.

At present, real time PCR is widely employed. In this technique, a serial dilution of the target nucleic acid is prepared, each thereof is subjected to PCR, and the time course is then taken in real time. The threshold cycle (the Ct value), with which a given amount of amplified product is obtained in a region where amplification exponentially occurs before reaching the level of the plateau effect, is determined. The determined value is plotted on a vertical axis, and the amount of nucleic acid is plotted on a horizontal axis. Thus, the calibration curve is prepared. An unknown sample of interest is subjected to PCR under the same conditions and the Ct value thereof is determined. This enables the quantification of the amount of nucleic acid in the unknown sample. A device for real time detection is disadvantageously expensive. If this technique is performed using a common commercialized thermal cycler, the sample has to be analyzed each cycle in order to determine the threshold cycle with which a given amount of amplified product is generated. Thus, this technique requires a large amount of labor.

Quantitative competitive PCR is also a widely employed technique. In this technique, a competitor nucleic acid having a sequence similar to that of the target nucleic acid is diluted in a stepwise manner, and the resultants are added to a sample containing the target nucleic acid to be quantified. Depending on the amount of the competitor nucleic acid added, the ratio of the amount of the amplified product from the target nucleic acid to the amount of the amplified product from competitor nucleic acid added, is determined. Accordingly, the point where the amount of the amplified product from target nucleic acid which was added becomes equal to the amount of the amplified product from competitor nucleic acid, represents the amount of the target nucleic acid. Although this technique is relatively simple, necessity of preparing competitors for each primer complicates the operation. In addition, there is a problem that the amplification efficiency of the target nucleic acid may differ from that of the competitor nucleic acid. Regarding the detection system, a system for assaying by-products of PCR, such as a system for detecting pyrophosphoric acid, can not be applied.

WO 02/18616 relates to modified template-mimic oligonucleotides (TMOs) used to adjust the amplification efficiency of an abundant target without affecting the amplification of other targets in a DNA synthesis reaction. The TMOs may have a 3' terminal modified to prevent its extension by DNA polymerase. Moreover, the 5' end base of each TMO is complementary to the 3' end base of the corresponding amplification oligo.

### Summary of the Invention

An object of the present invention is to provide a method for quantifying a target nucleic acid by PCR. More particularly, an object of the present invention is to provide a method for quantifying a target nucleic acid by PCR that utilizes a reaction system which was designed in such a way that the final amount of the amplified product reflects the amount of the target nucleic acid unlike conventional PCR techniques.

The present inventors have conducted concentrated studies in order to achieve the above objects. As a result, they have found that the final amount of the amplified product can reflect the amount of the target nucleic acid by conducting the polymerase chain reaction in the presence of a pair of competitive primers having sequences complementary to the sequences of the pair of amplification primers, wherein each competitive primer has, at its 5' terminus, a farther sequence of at least one or two nucleotide(s) that is not complementary to the target nucleic acid and which prevents the competitive primer from being congruous with the target nucleic acid. This has led to the completion of the present invention.

Thus, the present invention provides a method for quantifying a target nucleic acid by the polymerase chain reaction utilizing a template nucleic acid comprising a sequence of the target nucleic acid, a pair of primers for amplifying the target nucleic acid, and polymerase, wherein the polymerase chain reaction is conducted in the presence of a pair of competitive primers having sequences complementary to the sequences of the pair of amplification primers, wherein each competitive primer has, at its 5' terminus, a farther sequence of at least one or two nucleotide(s) that is not complementary to the target nucleic acid and which prevents the competitive primer from being congruous with the target nucleic acid.

Preferably, the ratio of the competitive primer to the amplification primer is 1:100 to 1:1.

Preferably, the 3' terminus of the competitive primer is modified in such a way that the competitive primer itself is not an initiation site for polymerase reaction.

Preferably, the 3' terminus of the competitive primer is phosphorylated, the nucleotide at the 3' terminus of the competitive primer is dideoxynucleotide, or the competitive primer has, at its 3' terminus, a sequence of at least one nucleotide that is not complementary to the target nucleic acid.

Preferably, the target nucleic acid in a sample is quantified by assaying the amount of the amplified product in a specified cycle in the polymerase reaction.

Preferably, the target nucleic acid in a sample is quantified by using a calibration curve that was prepared using a known amount of nucleic acid.

Preferably, the target nucleic acid is quantified by assaying the amplification product by electrophoresis, chromatography, or HPLC.

Preferably, the target nucleic acid is quantified by using a reaction by-product of the polymerase reaction.

Preferably, the reaction by-product is pyrophosphoric acid.

Preferably, pyrophosphoric acid is detected by using a dry analytical element.

Another aspect of the present invention provides a pair of competitive primers to be used in the method according to the present invention, which have sequences complementary to the pair of amplification primers used in the polymerase chain reaction.

Specifically, the pair of competitive primers has sequences complementary to the pair of amplification primers used in the polymerase chain reaction, wherein each competitive primer has, at its 5' terminus, a farther sequence of at least one or two nucleotide(s) that is not complementary to the target nucleic acid and which prevents the competitive primer from being congruous with the target nucleic acid.

Preferably, the 3' terminus of the competitive primer is modified in such a way that the competitive primer itself is not an initiation site for polymerase reaction. Particularly preferably, the 3' terminus of the competitive primer is phosphorylated, the nucleotide at the 3' terminus of the competitive primer is dideoxynucleotide, or the competitive primer has, at its 3' terminus, a sequence of at least one nucleotide that is not complementary to the target nucleic acid.

### Brief Description of the Drawings

Fig. 1 shows the correlation between the initial template amount in PCR and the optical density of reflection (ODR) 5 minutes later.
Fig. 2 shows the correlation between the initial template amount in PCR and the optical density of reflection (ODR) 5 minutes later.

### Best Mode for Carrying out the Invention

The method of the present invention is a method for quantifying a target nucleic acid by the polymerase chain reaction that utilizes a template nucleic acid comprising the target nucleic acid, a pair of primers for amplifying the target nucleic acid, and polymerase, which is characterized in that the polymerase chain reaction is conducted in the presence of a pair of competitive primers having sequences complementary to the sequences of the pair of amplification primers, wherein each competitive primer has, at its 5' terminus, a farther sequence of at least one or two nucleotide(s) that is not complementary to the target nucleic acid and which prevents the competitive primer from being congruous with the target nucleic acid.

In a preferable embodiment of the method for quantifying the target nucleic acid of the present invention, the target nucleic acid is detected or quantified by using pyrophosphoric acid that is a byproduct of the polymerase reaction. More preferably, pyrophosphoric acid is analyzed by colorimetry. Further preferably, pyrophosphoric acid is detected by using a dry analytical element.

The first preferred embodiment of the method according to the present invention is listed below.
(i) Detection of pyrophosphoric acid is carried out by using a dry analytical element for quantifying pyrophosphoric acid which comprises a reagent layer containing xanthosine or inosine, pyrophosphatase, purine nucleoside phosphorylase, xanthine oxidase, peroxidase, and a color developer.
(ii) Polymerase to be used is selected from the group consisting of DNA polymerase I, Klenow fragment of DNA polymerase I, Bst DNA polymerase, and reverse transcriptase.

The second preferred embodiment of the present invention is characterized in that the detection of pyrophosphoric acid which is generated upon the polymerase elongation reaction is carried out by enzymatically converting pyrophosphoric acid into inorganic phosphorous, followed by the use of a dry analytical element for quantifying inorganic phosphorus which comprises a reagent layer containing xanthosine or inosine, purine nucleoside phosphorylase, xanthine oxidase, peroxidase, and a color developer.

Preferred embodiments of a method according to the second aspect of the present invention are listed below.
(i) Pyrophosphatase is used as an enzyme for converting pyrophosphoric acid.
(ii) Polymerase to be used is selected from the group consisting of DNA polymerase I, Klenow fragment of DNA polymerase I, Bst DNA polymerase, and reverse transcriptase.

The embodiments of the present invention will be described in more detail in the following.

### (A) Target nucleic acid fragment:

A target nucleic acid to be analyzed in the present invention is polynucleotide, at least a part of its nucleotide sequence being known, and examples thereof include a genomic DNA fragment isolated from all the organisms including animals, microorganisms, bacteria, and plants, mRNA contained in cells, and cDNA fragment which is synthesized using mRNA as template. Also, RNA fragment or DNA fragment which can be isolated from virus can be a target nucleic acid. Preferably, the target nucleic acid fragment is purified as highly as possible, and an extra ingredient other than a nucleic acid fragment is removed. For example, when a genomic DNA fragment isolated from blood of animal (e.g., human) or nucleic acid (DNA or RNA) fragments of infectious bacteria or virus existing in blood are analyzed, cell membrane of leucocyte which was destructed in the isolation process, hemoglobin which was eluted from erythrocytes, and other general chemical substances in blood should be fully removed. In particular, hemoglobin inhibits the subsequent polymerase elongation reaction. Pyrophosphoric acid and phosphoric acid existing in blood as general biochemical substances are disturbing factors for accurate detection of pyrophosphoric acid generated by polymerase elongation reaction.

### (B) Primer complementary with target nucleic acid fragment (amplification primer for amplifying target nucleic acid):

A primer complementary with a target nucleic acid fragment used in the present invention is oligonucleotide having a nucleotide sequence complementary with a target site, the nucleotide sequence of the target nucleic acid fragment being known. Hybridization of a primer complementary with the target nucleic acid fragment to a target site of the target nucleic acid fragment results in progress on polymerase elongation reaction starting from the 3' terminus of the primer and using the target nucleic acid as template. Thus, whether or not the primer recognizes and specifically hybridizes to a target site of the target nucleic acid fragment is an important issue in the present invention. The number of nucleotides in the primer used in the present invention is preferably 5 to 60, and particularly preferably 15 to 40. If the number of nucleotides in the primer is too small, specificity with the target site of the target nucleic acid fragment is deteriorated and also a hybrid with the target nucleic acid fragment cannot be stably formed. When the number of nucleotides in the primer is too high, double-strands are disadvantageously formed due to hydrogen bonds between primers or between nucleotides in a primer. This also results in deterioration in specificity.

When the existence of the target nucleic acid fragment is detected by the method according to the present invention, a plurality of primers complementary with each different site in the target nucleic acid fragment can be used. Thus, recognition of the target nucleic acid fragment in a plurality of sites results in improvement in specificity in detecting the existence of the target nucleic acid fragment. When a part of the target nucleic acid fragment is amplified (e.g., PCR), a plurality of primers can be designed in accordance with the amplification methods.

When the nucleotide sequence of the target nucleic acid is detected by the method according to the present invention, particularly when the occurrence of mutation or polymorphisms is detected, a primer is designed in accordance with a type of nucleotide corresponding to mutation or polymorphisms so as to contain a portion of mutation or polymorphisms of interest. Thus, the occurrence of mutation or polymorphisms of the target nucleic acid fragment causes difference in the occurrence of hybridization of the primer to the target nucleic acid fragment, and the detection as difference in polymerase elongation reaction eventually becomes feasible. By setting a portion corresponding to mutation or polymorphisms around the 3' terminus of the primer, difference in recognition of the polymerase reaction site occurs, and this eventually enables the detection as difference in polymerase elongation reaction.

### (C) Competitive primer having a sequence complementary to the sequence of an amplification primer

In the present invention, the polymerase chain reaction is conducted in the presence of a pair of competitive primers having sequences complementary to the sequences of the aforementioned pair of amplification primers. The competitive primer has a sequence that is complementary to the sequence of the respective amplification primer and has, at its 5' terminus, a farther sequence of at least one or two nucleotide(s) that is not complementary to the target nucleic acid and which prevents the competitive primer from being congruous with the target nucleic acid. The competitive primer is preferably comprised of 5 to 60 nucleotides, and particularly preferably 15 to 40 nucleotides.

Preferably, the ratio of the competitive primer to the amplification primer is 1:100 to 1:1.

Preferably, the 3' terminus of the competitive primer is modified in such a way that the competitive primer itself is not an initiation site for polymerase reaction. Particularly preferably, (1) the 3' terminus of the competitive primer is phosphorylated, (2) the nucleotide at the 3' terminus of the competitive primer is dideoxynucleotide, or (3) the competitive primer has, at its 3' terminus, a sequence of at least one nucleotide that is not complementary to the target nucleic acid. In the case of (2) above, ddNTP that is used for sequencing is utilized, and ddNTP is used instead of dNTP on the 3' terminal side. This can prevent any further polymerase reaction from occurring. In the case of (3) above, a nucleotide that is not complementary to the target nucleic acid is added to a farther site of the 3' terminus. This can prevent the competitive primer from being congruous with the target nucleic acid.

### (D) Polymerase:

When the target nucleic acid is DNA, polymerase used in the present invention is DNA polymerase which catalyzes complementary elongation reaction which starts from the double-strand portion formed by hybridization of the primer with the target nucleic acid fragment in its portion denatured into single-strand in the 5' → 3' direction by using deoxynucleoside triphosphate (dNTP) as material and using the target nucleic acid fragment as template. Specific examples of DNA polymerase used include DNA polymerase I, Klenow fragment of DNA polymerase I, and Bst DNA polymerase. DNA polymerase can be selected or combined depending on the purpose. For example, when a part of the target nucleic acid fragment is amplified (e.g., PCR), use of Taq DNA polymerase which is excellent in heat resistance, is effective. When a part of the target nucleic acid fragment is amplified by using the amplification method (loop-mediated isothermal amplification of DNA (the LAMP method)) described in "BIO INDUSTRY, Vol. 18, No. 2, 2001," use of Bst DNA polymerase is effective as strand displacement-type DNA polymerase which has no nuclease activity in the 5' → 3' direction and catalyzes elongation reaction while allowing double-strand DNA to be released as single-strand DNA on the template. Use of DNA polymerase a, T4 DNA polymerase, and T7 DNA polymerase, which have hexokinase activity in the 3' → 5' direction in combination is also possible depending on the purpose.

When mRNA is a target nucleic acid fragment, reverse transcriptase having reverse transcription activity can be used. Further, reverse transcriptase can be used in combination with Taq DNA polymerase, or an enzyme having reverse transcriptase activity and DNA polymerase activity in combination may be used. These enzymes are used in the case of RT-PCR.

### (E) Polymerase elongation reaction:

Polymerase elongation reaction in the present invention includes all the elongation processes of complementary nucleic acids. These elongation processes proceed by starting from the 3' terminus of a primer complementary to the target nucleic acid fragment as described in (B) above, which was specifically hybridized to a part of the region of the target nucleic acid fragment which was denatured into a single strand as described in (A). Also, deoxynucleoside triphosphates (dNTP) are used as components, the polymerase as described in (C) above is used as a catalyst, and the target nucleic acid fragment is used as a template. This elongation reaction of complementary nucleic acids indicates that continuous elongation reaction occurs at least twice (corresponding to 2 nucleotides).

Examples of a representative polymerase elongation reaction and an amplification reaction of a subject site of the target nucleic acid fragment involving polymerase elongation reaction are shown below. The simplest case is that only one polymerase elongation reaction in the 5' → 3' direction is carried out using the target nucleic acid fragment as template. This polymerase elongation reaction can be carried out under isothermal conditions. In this case, the amount of pyrophosphoric acid generated as a result of polymerase elongation reaction is in proportion to the initial amount of the target nucleic acid fragment. Specifically, it is a suitable method for quantitatively detecting the existence of the target nucleic acid fragment.

When the amount of the target nucleic acid is small, a target site of the target nucleic acid is preferably amplified by any means utilizing polymerase elongation reaction. In the amplification of the target nucleic acid, various methods which have been heretofore developed, can be used. The most general and spread method for amplifying the target nucleic acid is polymerase chain reaction (PCR). PCR is a method of amplifying a target portion of the target nucleic acid fragment by repeating periodical processes of denaturing (a step of denaturing a nucleic acid fragment from double-strand to single-strand) → annealing (a step of hybridizing a primer to a nucleic acid fragment denatured into single-strand) → polymerase (Taq DNA polymerase) elongation reaction → denaturing, by periodically controlling the increase and decrease in temperature of the reaction solution. Finally, the target site of the target nucleic acid fragment can be amplified 1,000,000 times as compared to the initial amount. Thus, the amount of accumulated pyrophosphoric acid generated upon polymerase elongation reaction in the amplification process in PCR becomes large, and thereby the detection becomes easy.

A cycling assay method using exonuclease described in Japanese Patent Publication Laying-Open No. 5-130870 is a method for amplifying a target site of the target nucleic acid fragment utilizing polymerase elongation. In this method, a primer is decomposed from a reverse direction by performing polymerase elongation reaction starting from a primer specifically hybridized with a target site of the target nucleic acid fragment, and allowing 5' → 3' exonuclease to act. In place of the decomposed primer, a new primer is hybridized, and elongation reaction by DNA polymerase proceeds again. This elongation reaction by polymerase and the decomposition reaction by exonuclease for removing the previously elongated strand are successively and periodically repeated. The elongation reaction by polymerase and the decomposition reaction by exonuclease can be carried out under isothermal conditions. The amount of accumulated pyrophosphoric acid generated in polymerase elongation reaction repeated in this cycling assay method becomes large, and the detection becomes easy.

The LAMP method is a recently developed method for amplifying a target site of the target nucleic acid fragment. This method is carried out by using at least 4 types of primers, which complimentarily recognize at least 6 specific sites of the target nucleic acid fragment, and strand displacement-type Bst DNA polymerase, which has no nuclease activity in the 5' → 3' direction and which catalyzes elongation reaction while allowing the double-strand DNA on the template to be released as single-strand DNA. In this method, a target site of the target nucleic acid fragment is amplified as a special structure under isothermal conditions. The amplification efficiency of the LAMP method is high, and the amount of accumulated pyrophosphoric acid generated upon polymerase elongation reaction is very large, and the detection becomes easy.

When the target nucleic acid fragment is a RNA fragment, elongation reaction is carried out by using reverse transcriptase having reverse transcription activity and using the RNA strand as template. Further, RT-PCR can be utilized where reverse transcriptase is used in combination with Taq DNA polymerase, and reverse transcription (RT) reaction is carried out, followed by PCR. An enzyme having reverse transcription activity and DNA polymerase activity in combination can also be used here. Detection of pyrophosphoric acid generated in the RT reaction or RT-PCR reaction enables the detection of the existence of the RNA fragment of the target nucleic acid fragment. This method is useful for the detection of existence of RNA virus.

### (F) Detection of pyrophosphoric acid (PPi):

A method represented by formula 1 has been heretofore known as a method for detecting pyrophosphoric acid (PPi). In this method, pyrophosphoric acid (PPi) is converted into adenosinetriphosphate (ATP) with the aid of sulfurylase, and luminescence generated when adenosinetriphosphate acts on luciferin with the aid of luciferase is detected. Thus, an apparatus capable of measuring luminescence is required for detecting pyrophosphoric acid (PPi) by this method.

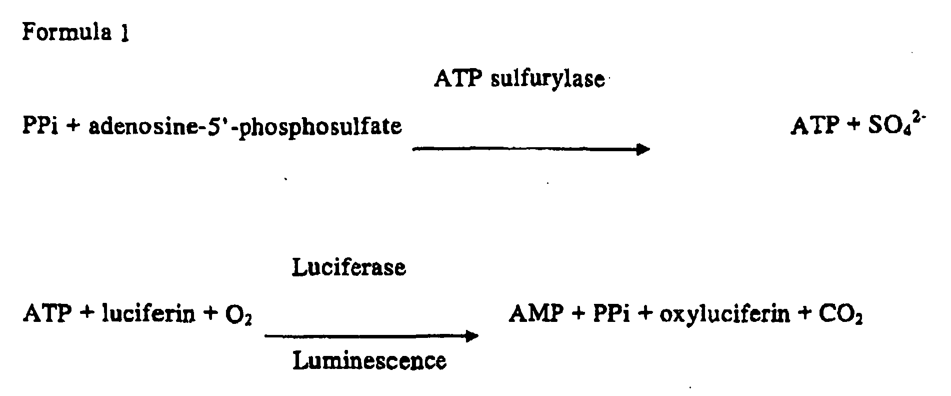

A method for detecting pyrophosphoric acid suitable for the present invention is a method represented by formula 2 or 3. In the method represented by formula 2 or 3, pyrophosphoric acid (PPi) is converted into inorganic phosphate (Pi) with the aid of pyrophosphatase, inorganic phosphate (Pi) is reacted with xanthosine or inosine with the aid of purine nucleoside phosphorylase (PNP), the resulting xanthine or hypoxanthine is oxidated with the aid of xanthine oxidase (XOD) to generate uric acid, and a color developer (a dye precursor) is allowed to develop color with the aid of peroxidase (POD) using hydrogen peroxide (H₂O₂) generated in the oxidation process, followed by colorimetry. In the method represented by formula 2 or 3, the result can be detected by colorimetry and, thus, pyrophosphoric acid (PPi) can be detected visually or using a simple colorimetric measuring apparatus.

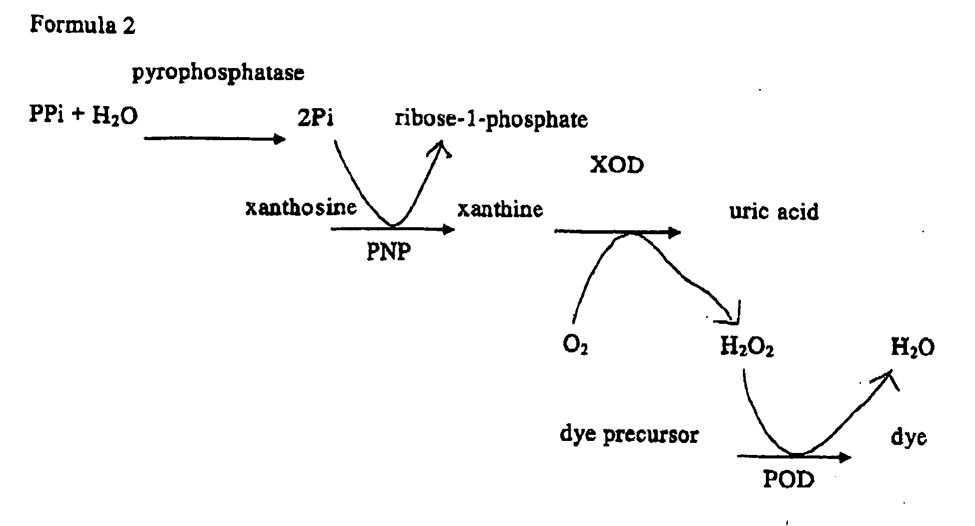

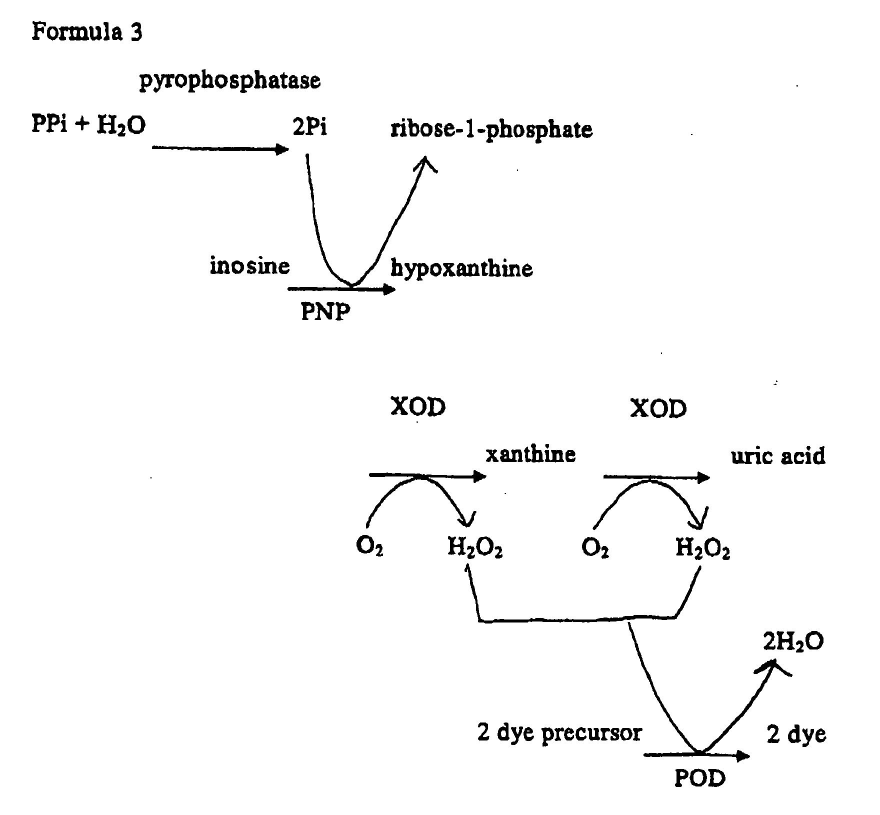

Commercially available pyrophosphatase (EC3, 6, 1, 1), purine nucleoside phosphorylase (PNP, EC2. 4. 2. 1), xanthine oxidase (XOD, EC1. 2. 3. 2), and peroxidase (POD, EC1. 11. 1. 7) can be used. A color developer (i.e., a dye precursor) may be any one as long as it can generate a dye by hydrogen peroxide and peroxidase (POD), and examples thereof which can be used herein include: a composition which generates a dye upon oxidation of leuco dye (e.g., triarylimidazole leuco dye described in U.S.Patent. No. 4,089,747 and the like, diarylimidazole leuco dye described in Japanese Patent Publication Laying-Open No. 59-193352 (EP 0122641A)); and a composition (e.g., 4-aminoantipyrines and phenols or naphthols) containing a compound generating a dye by coupling with other compound upon oxidation.

### (G) Dry analytical element:

A dry analytical element which can be used in the present invention is an analytical element which comprises a single or a plurality of functional layers, wherein at least one layer (or a plurality of layers) comprises a detection reagent, and a dye generated upon reaction in the layer is subjected to quantification by colorimetry by reflected light or transmitted light from the outside of the analytical element.

In order to perform quantitative analysis using such a dry analytical element, a given amount of liquid sample is spotted onto the surface of a developing layer. The liquid sample spread on the developing layer reaches the reagent layer and reacts with the reagent thereon and develops color. After spotting, the dry analytical element is maintained for a suitable period of time at given temperature (for incubation) and a color developing reaction is allowed to thoroughly proceed. Thereafter, the reagent layer is irradiated with an illuminating light from, for example, a transparent support side, the amount of reflected light in a specific wavelength region is measured to determine the optical density of reflection, and quantitative analysis is carried out based on the previously determined calibration curve.

Since a dry analytical element is stored and kept in a dry state before detection, it is not necessary that a reagent is prepared for each use. As stability of the reagent is generally higher in a dry state, it is better than a so-called wet process in terms of simplicity and swiftness since the wet process requires the preparation of the reagent solution for each use. It is also excellent as an examination method because highly accurate examination can be swiftly carried out with a very small amount of liquid sample.

### (H) Dry analytical element for quantifying pyrophosphoric acid:

A dry analytical element for quantifying pyrophosphoric acid which can be used in the present invention can have a layer construction which is similar to various known dry analytical elements. The dry analytical element may be multiple layers which contain, in addition to a reagent for performing the reaction represented by formula 2 or 3 according to item (F) above (detection of pyrophosphoric acid (PPi)), a support, a developing layer, a detection layer, a light-shielding layer, an adhesive layer, a water-absorption layer, an undercoating layer, and other layers. Examples of such dry analytical elements include those disclosed in the specifications of Japanese Patent Publication Laying-Open No. 49-53888 (U.S. Patent, No. 3,992,158), Japanese Patent Publication Laying-Open No. 51-40191 (U.S. Patent. No. 4,042,335), Japanese Patent Publication Laying-Open No. 55-164356 (U.S.Patent. No. 4,292,272), and Japanese Patent Publication Laying-Open No. 61-4959 (EPC Publication No. 0166365A).

Examples of the dry analytical element to be used in the present invention include a dry analytical element for quantifying pyrophosphoric acid which comprises a reagent for converting pyrophosphoric acid into inorganic phosphorus and a reagent layer containing a group of reagent for carrying out a coloring reaction depending of the amount of inorganic phosphorus.

In this dry analytical element for quantitative assay of pyrophosphate, pyrophosphoric acid (PPi) can enzymatically be converted into inorganic phosphorus (Pi) using pyrophosphatase as described above. The subsequent process, that is color reaction depending on the amount of inorganic phosphorus (Pi), can be performed using "quantitative assay method of inorganic phosphorus" (and combinations of individual reactions used therefor), described hereinafter, which is known in the field of biochemical inspection.

It is noted that when representing "inorganic phosphorus," both the expressions "Pi" and "HPO₄²⁻, H₂PO₄¹⁻" are used for phosphoric acid (phosphate ion). Although the expression "Pi" is used in the examples of reactions described below, the expression "HPO₄²⁻" may be used for the same reaction formula.

As the quantitative assay method of inorganic phosphorus, an enzyme method and a phosphomolybdate method are known. Hereinafter, this enzyme method and phosphomolybdate method will be described as the quantitative assay method of inorganic phosphorus.

### A. Enzyme method

Depending on the enzyme to be used for the last color reaction during a series of reactions for Pi quantitative detection, the following methods for quantitative assay are available: using peroxidase (POD); or using glucose-6-phosphate dehydrogenase (G6PDH), respectively. Hereinafter, examples of these methods are described.

### (1) Example of the method using peroxidase (POD)

### (1-1)

Inorganic phosphorus (Pi) is allowed to react with inosine by purine nucleoside phosphorylase (PNP), and the resultant hypoxanthine is oxidized by xanthine oxidase (XOD) to produce uric acid. During this oxidization process, hydrogen peroxide (H₂O₂) is produced. Using the thus produced hydrogen peroxide, 4-aminoantipyrines (4-AA) and phenols are subjected to oxidization-condensation by peroxidase (POD) to form a quinonimine dye, which is colorimetrically assessed.

### (1-2)

Pyruvic acid is oxidized by pyruvic oxidase (POP) in the presence of inorganic phosphorus (Pi), cocarboxylase (TPP), flavin adenine dinucleotide (FAD) and Mg²⁺ to produce acetyl acetate. During this oxidization process, hydrogen peroxide (H₂O₂) is produced. Using the thus produced hydrogen peroxide, 4-aminoantipyrines (4-AA) and phenols are subjected to oxidization-condensation by peroxidase (POD) to form a quinonimine dye which is colorimetrically assessed, in the same manner as described in (1-1).

It is noted that the last color reaction for each of the above processes (1-1) and (1-2) can be performed by a "Trinder reagent" which is known as a detection reagent for hydrogen peroxide. In this reaction, phenols function as "hydrogen donors." Phenols to be used as "hydrogen donors" are classical, and now various modified "hydrogen donors" are used. Examples of these hydrogen donors include N-ethyl-N-sulfopropyl-m-anilidine, N-ethyl-N-sulfopropylaniline, N-ethyl-N-sulfopropyl-3,5-dimethoxyaniline, N-sulfopropyl-3,5-dimethoxyaniline, N-ethyl-N-sulfopropyl-3,5-dimethylaniline, N-ethyl-N-sulfopropyl-m-toluidine, N-ethyl-N-(2-hydroxy-3-sulfopropyl)-m-anilidine N-ethyl-N-(2-hydroxy-3-sulfopropyl)aniline, N-ethyl-N-(2-hydroxy-3-sulfopropyl)-3,5-dimethoxyaniline, N-(2-hydroxy-3-sulfopropyl)-3,5-dimethoxyaniline, N-ethyl-N-(2-hydroxy-3-sulfopropyl)-3,5-dimethylaniline, N-ethyl-N-(2-hydroxy-3-sulfopropyl)-m-toluidine, and N-sulfopropylaniline.

### (2) Example of a method using glucose-6-phosphate dehydrogenase (G6PDH)

### (2-1)

Inorganic phosphorus (Pi) is reacted with glycogen with phosphorylase to produce glucose-1-phosphate (G-1-P). The produced glucose-1-phosphate is converted into glucose-6-phosphate (G-6-P) with phosphoglucomutase (PGM). In the presence of glucose-6-phosphate and nicotiamide adenine dinucleotide (NAD), NAD is reduced to NADH with glucose-6-phosphate dehydrogenase (G6PDH), followed by colorimetric analysis of the produced NADH.

### (2-2)

Inorganic phosphorus (Pi) is reacted with maltose with maltose phosphorylase (MP) to produce glucose-1-phosphate (G-1-P). Thereafter, the produced glucose-1-phosphate is converted into glucose-6-phosphate (G-6-P) with phosphoglucomutase (PGM) in the same manner as described in (2-1). In the presence of glucose-6-phosphate and nicotiamide adenine dinucleotide (NAD), NAD is reduced to NADH with glucose-6-phosphate dehydrogenase (G6PDH), followed by colorimetric analysis of the produced NADH.

### B. Phosphomolybdate method

There are two phosphomolybdate methods. One is a direct method wherein "Phosphomolybdates (H₃[PO₄Mo₁₂O₃₆])" prepared by complexing inorganic phosphorus (phosphate) and aqueous molybdate ions under acidic condition are directly quantified. The other is a reduction method wherein further to the above direct method, Mo(IV) is reduced to Mo(III) by a reducing agent and molybudenum blue (Mo(III)) is quantified. Examples of the aqueous molybdate ions include aluminum molybdate, cadmium molybdate, calcium molybdate, barium molybdate, lithium molybdate, potassium molybdate, sodium molybdate, and ammonium molybdate. Representative examples of the reducing agents to be used in the reduction method include 1-amino-2-naphthol-4-sulfonic acid, ammonium ferrous sulfate, ferrous chloride, stannous chloride-hydrazine, *p*-methylaminophenol sulfate, N,N-dimethyl-phenylenediamine, ascorbic acid, and malachite green.

When a light-transmissive and water-impervious support is used, the dry analytical element can be practically constructed as below. However, the scope of the present invention is not limited to these.
(1) One having a reagent layer on the support.
(2) One having a detection layer and a reagent layer in that order on the support.
(3) One having a detection layer, a light reflection layer, and a reagent layer in that order on the support.
(4) One having a second reagent layer, a light reflection layer, and a first reagent layer in that order on the support.
(5) One having a detection layer, a second reagent layer, a light reflection layer, and a first reagent layer in that order on the support.

In (1) to (3) above, the reagent layer may be constituted by a plurality of different layers. For example, a first reagent layer may contain enzyme pyrophosphatase which is required in the pyrophosphatase reaction represented by formula 2 or 3, and substrate xanthosine or substrate inosine and enzyme PNP which are required in the PNP reaction, a second reagent layer may contain enzyme XOD which is required in the XOD reaction represented by formula 2 or 3, and a third reagent layer may contain enzyme POD which is required in the POD reaction represented by formula 2 or 3, and a coloring dye (dye precursor). Alternatively, two reagent layers are provided. On the first reagent layer, the pyrophosphatase reaction and the PNP reaction may be proceeded, and the XOD reaction and the POD reaction may be proceeded on the second reagent layer. Alternatively, the pyrophosphatase reaction, the PNP reaction and the XOD reaction may be proceeded on the first reagent layer, and the POD reaction may be proceeded on the second reagent layer.

A water absorption layer may be provided between a support and a reagent layer or detection layer. A filter layer may be provided between each layer. A developing layer may be provided on the reagent layer and an adhesive layer may be provided therebetween.

Any of light-nontransmissive (opaque), light-semitransmissive (translucent), or light-transmissive (transparent) support can be used. In general, a light-transmissive and water-impervious support is preferred. Preferable materials for a light-transmissive and water-impervious support are polyethylene terephthalate or polystyrene. In order to firmly adhere a hydrophilic layer, an undercoating layer is generally provided or hydrophilization is carried out.

When a porous layer is used as a reagent layer, the porous medium may be a fibrous or nonfibrous substance. Fibrous substances used herein include, for example, filter paper, non-woven fabric, textile fabric (e.g. plain-woven fabric), knitted fabric (e.g., tricot knitted fabric), and glass fiber filter paper. Nonfibrous substances may be any of a membrane filter comprising cellulose acetate etc., described in Japanese Patent Publication Laying-Open No. 49-53888 and the like, or a particulate structure having mutually interconnected spaces comprising fine particles of inorganic substances or organic substances described in, for example, Japanese Patent Publication Laying-Open No. 49-53888, Japanese Patent Publication Laying-Open No. 55-90859 (U.S. Patent. No. 4,258,001), and Japanese Patent Publication Laying-Open No. 58-70163 (U.S. Patent. No. 4,486,537). A partially-adhered laminate which comprises a plurality of porous layers described in, for example, Japanese Patent Publication Laying-Open No. 61-4959 (EP Publication 0166365A), Japanese Patent Publication Laying-Open No. 62-116258, Japanese Patent Publication Laying-Open No. 62-138756 (EP Publication 0226465A), Japanese Patent Publication Laying-Open No. 62-138757 (EP Publication 0226465A), and Japanese Patent Publication Laying-Open No. 62-138758 (EP Publication 0226465A), is also preferred.

A porous layer may be a developing layer having so-called measuring action, which spreads liquid in an area substantially in proportion to the amount of the liquid to be supplied. Preferably, a developing layer is textile fabric, knitted fabric, and the like. Textile fabrics and the like may be subjected to glow discharge treatment as described in Japanese Patent Publication Laying-Open No. 57-66359. A developing layer may comprise hydrophilic polymers or surfactants as described in Japanese Patent Publication Laying-Open No. 60-222770 (EP 0162301A), Japanese Patent Publication Laying-Open No. 63-219397 (German Publication DE 3717913A), Japanese Patent Publication Laying-Open No. 63-112999 (DE 3717913A), and Japanese Patent Publication Laying-Open No. 62-182652 (DE 3717913A) in order to regulate a developing area, a developing speed and the like.

For example, a method is useful where the reagent of the present invention is previously impregnated into or coated on a porous membrane etc., comprising paper, fabric or polymer, followed by adhesion onto another water-pervious layer provided on a support (e.g., a detection layer) by the method as described in Japanese Patent Publication Laying-Open No. 55-1645356.

The thickness of the reagent layer thus prepared is not particularly limited. When it is provided as a coating layer, the thickness is suitably in the range of about 1 µm to 50 µm, preferably in the range of 2 µm to 30 µm. When the reagent layer is provided by a method other than coating, such as lamination, the thickness can be significantly varied in the range of several tens of to several hundred µm.

When a reagent layer is constituted by a water-pervious layer of hydrophilic polymer binders, examples of hydrophilic polymers which can be used include: gelatin and a derivative thereof (e.g., phthalated gelatin); a cellulose derivative (e.g., hydroxyethyl cellulose); agarose, sodium arginate; an acrylamide copolymer or a methacrylamide copolymer (e.g., a copolymer of acrylamide or methacrylamide and various vinyl monomers); polyhydroxyethyl methacrylate; polyvinyl alcohol; polyvinyl pyrrolidone; sodium polyacrylate; and a copolymer of acrylic acid and various vinyl monomers.

A reagent layer composed of hydrophilic polymer binders can be provided by coating an aqueous solution or water dispersion containing the reagent composition of the present invention and hydrophilic polymers on the support or another layer such as a detection layer followed by drying the coating in accordance with the methods described in the specifications of Japanese Patent Examined Publication No. 53-21677 (U.S. Patent No. 3,992,158), Japanese Patent Publication Laying-Open No. 55-164356 (U.S. Patent. No. 4,292,272), Japanese Patent Publication Laying-Open No. 54-101398 (U.S. Patent. No. 4,132,528) and the like. The thickness of the reagent layer comprising hydrophilic polymers as binders is about 2 µm to about 50 pm, preferably about 4 µm to about 30 µm on a dry basis, and the coverage is about 2 g/m² to about 50 g/m², preferably about 4 g/m² to about 30 g/m².

The reagent layer can further comprise an enzyme activator, a coenzyme, a surfactant, a pH buffer composition, an impalpable powder, an antioxidant, and various additives comprising organic or inorganic substances in addition to the reagent composition represented by formula 2 or 3 in order to improve coating properties and other various properties of diffusible compounds such as diffusibility, reactivity, and storage properties. Examples of buffers which can be contained in the reagent layer include pH buffer systems described in "Kagaku Binran Kiso (Handbook on Chemistry, Basic)," The Chemical Society of Japan (ed.), Maruzen Co., Ltd. (1996), p.1312-1320, "Data for Biochemical Research, Second Edition, R. M. C. Dawson et al. (2nd ed.), Oxford at the Clarendon Press (1969), p. 476-508, "Biochemistry" 5, p. 467-477 (1966), and "Analytical Biochemistry" 104, p. 300-310 (1980). Specific examples of pH buffer systems include a buffer containing borate; a buffer containing citric acid or citrate; a buffer containing glycine, a buffer containing bicine; a buffer containing HEPES; and Good's buffers such as a buffer containing MES. A buffer containing phosphate cannot be used for a dry analytical element for detecting pyrophosphoric acid.

The dry analytical element for quantifying pyrophosphoric acid which can be used in the present invention can be prepared in accordance with a known method disclosed in the above-described various patent specifications. The dry analytical element for quantifying pyrophosphoric acid is cut into small fragments, such as, an about 5 mm to about 30 mm-square or a circle having substantially the same size, accommodated in the slide frame described in, for example, Japanese Patent Examined Publication No. 57-283331 (U.S. Patent. No. 4,169,751), Japanese Utility Model Publication Laying-Open No. 56-142454 (U.S. Patent. No. 4,387,990), Japanese Patent Publication Laying-Open No. 57-63452, Japanese Utility Model Publication Laying-Open No. 58-32350, and Japanese Patent Publication Laying-Open No. 58-501144 (International Publication WO 083/00391), and used as slides for chemical analysis. This is preferable from the viewpoints of production, packaging, transportation, storage, measuring operation, and the like. Depending on its intended use, the analytical element can be accommodated as a long tape in a cassette or magazine, as small pieces accommodated in a container having an opening, as small pieces applied onto or accommodated in an open card, or as small pieces cut to be used in that state.

The dry analytical element for quantifying pyrophosphoric acid which can be used in the present invention can quantitatively detect pyrophosphoric acid which is a test substance in a liquid sample, by operations similar to that described in the above-described patent specifications and the like. For example, about 2 µL to about 30 µL, preferably 4 µL to 15 µL of aqueous liquid sample solution is spotted on the reagent layer. The spotted analytical element is incubated at constant temperature of about 20°C to about 45°C, preferably about 30°C to about 40°C for 1 to 10 minutes. Coloring or discoloration in the analytical element is measured by the reflection from the light-transmissive support side, and the amount of pyrophosphoric acid in the specimen can be determined based on the principle of colorimetry using the previously prepared calibration curve. Quantitative analysis can be carried out with high accuracy by keeping the amount of liquid sample to be spotted, the incubation time, and the temperate at constant levels.

Quantitative analysis can be carried out with high accuracy in a very simple operation using chemical analyzers described in, for example, Japanese Patent Publication Laying-Open No. 60-125543, Japanese Patent Publication Laying-Open No. 60-220862, Japanese Patent Publication Laying-Open No. 61-294367, and Japanese Patent Publication Laying-Open No. 58-161867 (U.S. Patent. No. 4,424,191). Semiquantitative measurement may be carried out by visually judging the level of coloring depending on the purpose and accuracy needed.

Since the dry analytical element for quantifying pyrophosphoric acid which can be used in the present invention is stored and kept in a dry state before analysis, it is not necessary that a reagent is prepared for each use, and stability of the reagent is generally higher in a dry state. Thus, in terms of simplicity and swiftness, it is better than a so-called wet process, which requires the preparation of the reagent solution for each use. It is also excellent as an examination method because highly accurate examination can be swiftly carried out with a very small amount of liquid sample.

The dry analytical element for quantifying inorganic phosphorus which can be used in the second aspect of the present invention can be prepared by removing pyrophosphatase from the reagent layer in the aforementioned dry analytical element for quantifying pyrophosphoric acid. The dry analytical element described in Japanese Patent Publication Laying-Open No. 7-197 can also be used. The dry analytical element for quantifying inorganic phosphorus is similar to the aforementioned dry analytical element for quantifying pyrophosphoric acid in its layer construction, method of production, and method of application, with the exception that the reagent layer does not comprise pyrophosphatase.

The present invention is described in more detail with reference to the following examples. However, the technical scope of the present invention is not limited by these examples.

### Examples

### Example 1: Quanification of initial template amount by PCR utilizing a pyrophosphoric acid slide, and preparation of a calibration curve

### (1) Preparation of a plasmid for quantification

Plasmid (pBluescript vector) comprising β-actin gene (about 1500 bp) inserted therein was introduced into an *E*. *coli* competent cell (JM 109). The resultant was cultured in LB medium overnight. Plasmid was extracted and purified therefrom to obtain a plasmid comprising β-actin gene fragment inserted therein. Further, the amount of the obtained plasmid was quantified by using a spectrophotometer, and solutions respectively containing 0.1 ng/µl, 3 pg/µl, 0.1 pg/µl, 3 fg/µl, and 0.1 fg/µl of the plasmid were prepared.

### (2) Amplification by PCR

Amplification by PCR was conducted under the following conditions using known amount of plasmid prepared in (1) above.

### <Primers>

The following primer set having a sequence specific to the β-actin gene fragment was used.
Primer (upper): 5'-GGGCATGGGTCAGAAGGATT-3' (SEQ ID NO: 1)
Primer (lower): 5'-CCGTGGTGGTGAAGCTGTAG-3' (SEQ ID NO: 2)

The following primer pair (CP2) having its 3' termini being phosphorylated and having sequences complementary to the respective sequences of the above primers was used as a primer pair for quantification.
CP2 Primer (upper): 5'-TTAATCCTTCTGACCCATGCCC-3' (SEQ ID NO: 3)
CP2 Primer (lower): 5'-TTCTACAGCTTCACCACCACGG-3' (SEQ ID NO: 4)

An underlined portion indicates a portion that is complementary to each primer.

Six levels of solutions initially containing 0, 0.1 fg, 3 fg, 0.1 pg, 3 pg, and 0.1 ng of nucleic acids were prepared in the following compositions for each of two lines, i.e., a line comprising a CP primer pair and that containing no CP primer pair. Amplification by PCR was conducted.

| Table 1: Composition of reaction solution | Line 1 | Line 2 (control) |
|---|---|---|
| 10× PCR buffer | 5 µl | 5 µl |
| 2.5 mM dNTP | 5 µl | 5 µl |
| 5 µM primer (upper) | 2.5 µl | 2.5 µl |
| 5 µM primer (lower) | 2.5 µl | 2.5 µl |
| 0.5 µM CP primer (upper) | 2.5 µl | - |
| 0.5 µM CP primer (lower) | 2.5 µl | - |
| HS Taq (TaKaRa) | 0.5 µl | 0.5 µl |
| Each nucleic acid solution obtained in (1) | 1 µl | 1 µl |
| Purified water | 28.5 µl | 33.5 µl |

Amplification by PCR was carried out by repeating 35 cycles of denaturing at 94°C for 20 seconds, annealing at 60°C for 30 seconds, and polymerase elongation at 72°C for 1 minute.

### (3) Preparation of a dry analytical element for pyrophosphoric acid quantification

A colorless transparent polyethylene terephthalate (PET) smooth film sheet (support) comprising a gelatin undercoating layer (thickness of 180 µm) was coated with an aqueous solution having composition (a) shown in Table 2 to the following coverage. The coating was then dried to provide a reagent layer.

**Table 2: Composition (a) of aqueous solution for reagent layer**

| | |
|---|---|
| Gelatin | 18.8 g/m² |
| p-Nonylphenoxy polyxydol | 1.5 g/m² |
| (containing 10 glycidol units on average) | |
| (C₉H₁₉-Ph-O-(CH₂CH(OH)-CH₂-O)₁₀H) | |
| Xanthosine | 1.96 g/m² |
| Peroxidase | 15,000 IU/m² |
| Xanthine oxidase | 13,600 IU/m² |
| Purine nucleoside phosphorylase | 3,400 IU/m² |
| Leuco dye | 0.28 g/m² |
| (2-(3,5-dimethoxy-4-hydroxyphenyl)-4- phenethyl-5-(4-dimethylaminophenyl)imidazole) | |
| Water | 136 g/m² |
| (pH was adjusted to 6.8 with a diluted NaOH solution) | |

This reagent layer was coated with an aqueous solution for an adhesive layer having composition (b) shown in Table 3 below to the following coverage. The coating was then dried to provide an adhesive layer.

**Table 3: Composition (b) of aqueous solution for adhesive layer**

| | |
|---|---|
| Gelatin | 3.1 g/m² |
| p-Nonylphenoxy polyxydol | 0.25 g/m² |
| (containing 10 glycidol units on average) | |
| (C₉H₁₉-Ph-O-(CH₂CH(OH)-CH₂-O)₁₀H) | |
| Water | 59 g/m² |

Subsequently, water was supplied to the adhesive layer over its whole surface at 30 g/m² to allow the gelatin layer to swell. A broad textile fabric made of genuine polyester was laminated thereon by applying slight pressure in a substantially even manner to provide a porous developing layer.

The developing layer was then substantially evenly coated with an aqueous solution having composition (c) shown in Table 4 below to the following coverage. The coating was then dried, cut into a size of 13 mm x 14 mm, and accommodated into a plastic mounting material, thereby preparing a dry analytical element for pyrophosphoric acid quantification.

**Table 4: Composition (c) of aqueous solution for developing layer**

| | |
|---|---|
| HEPES | 2.3 g/m² |
| Sucrose | 5.0g/m² |
| Hydroxypropyl methylcellulose | 0.04 g/m² |
| (methoxy group 19% to 24%, hydroxypropoxy group 4% to 12%) | |
| Pyrophosphatase | 14,000 IU/m² |
| Water | 98.6 g/m² |
| (pH was adjusted to 7.2 with a diluted NaOH solution) | |

### (4) Detection using an analytical element for pyrophosphoric acid quantification

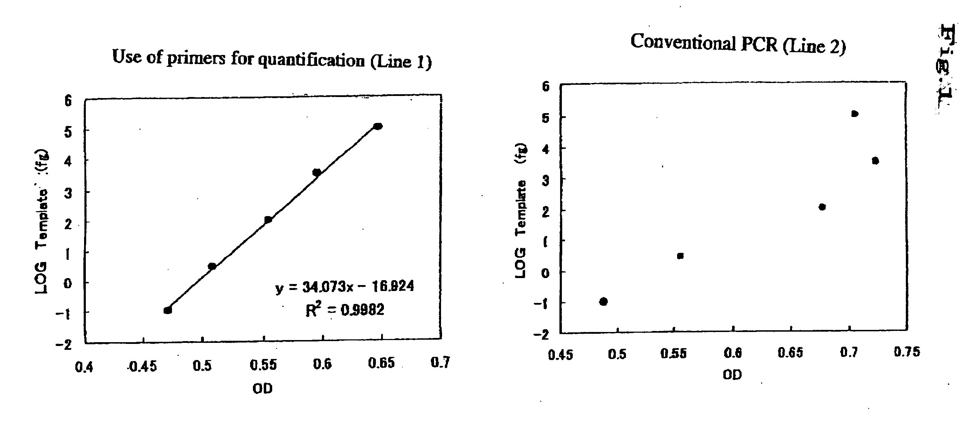

The solutions after amplification by PCR in (2) above were spot deposited as they were on the dry analytical element for pyrophosphoric acid quantification prepared in (3) above in amounts of 20 µl each, and the dry analytical element for pyrophosphoric acid quantification was incubated at 37°C for 5 minutes. Thereafter, the optical density of reflection (OD_{R}) was assayed at the wavelength of 650 nm from the support side. The results are shown in Table 5. Fig. 1 shows a chart for the calibration curve of OD_{R} relative to the exponent of the initial amount in PCR obtained from Table 5.

**Table 5: Correlation between the initial template amount in PCR and the optical density of reflection (OD_{R}) 5 minutes later**

| Initial template amount | Optical density of reflection (OD_{R}) | 5 minutes later |
|---|---|---|
| (fg/50 µl) | Line 1 | Line 2 |
| 0 | 0.440 | 0.442 |
| 0.1 | 0.470 | 0.488 |
| 3 x 10 | 0.509 | 0.555 |
| 1 x 10³ | 0.555 | 0.678 |
| 3 x 10⁴ | 0.591 | 0.725 |
| 1 x 10⁶ | 0.647 | 0.706 |

As is apparent from the results obtained in Example 1, no correlation is observed between the initial template amount and ODR in conventional PCR (line 2). In contrast, when a primer pair (CP2) that is partially complementary to the primer pair for PCR is used, there is correlation between the exponent of the initial template amount and ODR. Thus, the initial template amount can be quantified by carrying out PCR that involves the CP2 primer pair with the use of this calibration curve.

### Example 2: Quantification of an unknown initial template amount by PCR using a pyrophosphoric acid slide

### (1) Preparation of a plasmid for quantification

Solutions containing 10 pg/µl or 10 fg/µl of nucleic acid were prepared by using the plasmid prepared in Example 1 (1), and PCR was carried out in the same manner as in Example 1. Whether or not the initial template amount was accurately quantified from the calibration curve obtained therein was examined.

### (2) Amplification by PCR

PCR was conducted for each template amount by the method described in Example 1.

### (3) Preparation of a dry analytical element for pyrophosphoric acid quantification

The dry analytical element for pyrophosphoric acid was prepared in the manner described in Example 1.

### (4) Detection using an analytical element for pyrophosphoric acid quantification

The solutions after amplification by PCR in (2) above were spotted as they were on the dry analytical element for pyrophosphoric acid quantification prepared in (3) above in amounts of 20 µL each, and the dry analytical element for pyrophosphoric acid quantification was incubated at 37°C for 5 minutes. Thereafter, the optical density of reflection (ODR) was assayed at the wavelength of 650 nm from the support side. The results are shown in Table 6. The ODR value obtained from Table 6 was rearranged using the chart for the calibration curve in Example 1 to determine the initial DNA amount. The results are shown in Table 7.

**Table 6: Correlation between the initial template amount in PCR and the optical density of reflection (ODR) 5 minutes later.**

| Initial template amount | Optical density of reflection (ODR) | 5 minutes later |
|---|---|---|
| (fg/50 µl) | Line 1 | Line 2 |
| 0 | 0.448 | 0.445 |
| 10 | 0.520 | 0.603 |
| 1 x 10⁴ | 0.605 | 0.646 |

**Table 7: The initial template amount in PCR rearranged from Example 1**

| Initial template amount | Initial template amount calculated from the calibration curve (fg/50 µl) | |
|---|---|---|
| (fg/50 µl) | Line 1 | Line 2 |
| 0 | 0.02 | |
| 10 | 6.2 | - |
| 1 x 10⁴ | 4.9 x 10³ | - |

As is apparent from the results obtained in Example 2, the obtained values are very close to the initial template amount that was first calculated. Thus, the initial template amount of PCR can be determined by using a CP2 primer pair.

### Example 3: Quantification of initial template amount by PCR utilizing a pyrophosphoric acid slide, and preparation of a calibration curve

### (1) Preparation of a plasmid for quantification

Plasmid (pBluescript vectors) comprising β-actin gene (about 1500 bp) inserted therein was introduced into an *E*. *coli* competent cell (JM 109). The resultant was cultured in LB medium overnight. Plasmid was extracted and purified therefrom to obtain a plasmid comprising β-actin gene fragment inserted therein. Further, the amount of the obtained plasmid was quantified by using a spectrophotometer, and solutions respectively containing 0.1 ng/µl, 3 pg/µl, 0.1 pg/µl, 3 fg/µl, and 0.1 fg/µl of the plasmid were prepared.

### (2) Amplification by PCR

Amplification by PCR was conducted under the following conditions using known amount of plasmid prepared in (1) above.

### <Primers>

The following primer set having a sequence specific to the β-actin gene fragment was used.
Primer (upper): 5'-GGGCATGGGTCAGAAGGATT-3' (SEQ ID NO: 5)
Primer (lower): 5'-CCGTGGTGGTGAAGCTGTAG-3' (SEQ ID NO: 6)

The following primer pair (CP0) having its 3' termini being phosphorylated and having sequences complementary to the respective sequence of the above primers was used as a primer pair for quantification.
CP0 Primer (upper): 5'-AATCCTTCTGACCCATGCCC-3' (SEQ ID NO: 7)
CP0 Primer (lower): 5'-CTACAGCTTCACCACCACGG-3' (SEQ ID NO: 8)

Six levels of solutions initially containing 0, 0.1 fg, 3 fg, 0.1 pg, 3 pg, and 0.1 ng of nucleic acids were prepared in the following compositions for each of two lines, i.e., a line comprising the CP0 primer pair and that containing no CP0 primer pair. Amplification by PCR was conducted.

| Table 8: Composition of reaction solution | Line 1 | Line 2 (control) |
|---|---|---|
| 10× PCR buffer | 5 µl | 5 µl |
| 2.5 mM dNTP | 5 µl | 5 µl |
| 5 µM primer (upper) | 2.5 µl | 2.5 µl |
| 5 µM primer (lower) | 2.5 µl | 2.5 µl |
| 0.5 µM CP0 primer (upper) | 2.5 µl | - |
| 0.5 µM CP0 primer (lower) | 2.5 µl | - |
| HS Taq (TaKaRa) | 0.5 µl | 0.5 µl |
| Each nucleic acid solution obtained in (1) | 1 µl | 1 µl |
| Purified water | 28.5 µl | 33.5 µl |

Amplification by PCR was carried out by repeating 35 cycles of denaturing at 94°C for 20 seconds, annealing at 60°C for 30 seconds, and polymerase elongation at 72°C for 1 minute.

### (3) Preparation of a dry analytical element for pyrophosphoric acid quantification

A colorless transparent polyethylene terephthalate (PET) smooth film sheet (support) comprising a gelatin undercoating layer (thickness of 180 µm) was coated with an aqueous solution having composition (a) shown in Table 9 to the following coverage. The coating was then dried to provide a reagent layer.

**Table 9: Composition (a) of aqueous solution for reagent layer**

| | |
|---|---|
| Gelatin | 18.8 g/m² |
| p-Nonylphenoxy polyxydol | 1.5 g/m² |
| (containing 10 glycidol units on average) | |
| (C₉H₁₉-Ph-O-(CH₂CH(OH)-CH₂-O)₁₀H) | |
| Xanthosine | 1.96 g/m² |
| Peroxidase | 15,000 IU/m² |
| Xanthine oxidase | 13,600 IU/m² |
| Purine nucleoside phosphorylase | 3,400 IU/m² |
| Leuco dye | 0.28 g/m² |
| (2-(3,5-dimethoxy-4-hydroxyphenyl)-4- phenethyl-5-(4-dimethylaminophenyl)imidazole) | |
| Water | 136 g/m² |
| (pH was adjusted to 6.8 with a diluted NaOH solution) | |

This reagent layer was coated with an aqueous solution for an adhesive layer having composition (b) shown in Table 10 below to the following coverage. The coating was then dried to provide an adhesive layer.

**Table 10: Composition (b) of aqueous solution for adhesive layer**

| | |
|---|---|
| Gelatin | 3.1 g/m² |
| p-Nonylphenoxy polyxydol | 0.25 g/m² |
| (containing 10 glycidol units on average) | |
| (C₉H₁₉-Ph-O-(CH₂CH(OH)-CH₂-O)₁₀H) | |
| Water | 59 g/m² |

Subsequently, water was supplied to the adhesive layer over its whole surface at 30 g/m² to allow the gelatin layer to swell. A broad textile fabric made of genuine polyester was laminated thereon by applying slight pressure in a substantially even manner to provide a porous developing layer.

The developing layer was then substantially evenly coated with an aqueous solution having composition (c) shown in Table 11 below to the following coverage. The coating was then dried, cut into a size of 13 mm x 14 mm, and accommodated into a plastic mounting material, thereby preparing a dry analytical element for pyrophosphoric acid quantification.

**Table 11: Composition (c) of aqueous solution for developing layer**

| | |
|---|---|
| HEPES | 2.3 g/m² |
| Sucrose | 5.0g/m² |
| Hydroxypropyl methylcellulose | 0.04 g/m² |
| (methoxy group 19% to 24% hydroxypropoxy group 4% to 12%) | |
| Pyrophosphatase | 14,000 IU/m² |
| Water | 98.6 g/m² |
| (pH was adjusted to 7.2 with a diluted NaOH solution) | |

### (4) Detection using an analytical element for pyrophosphoric acid quantification

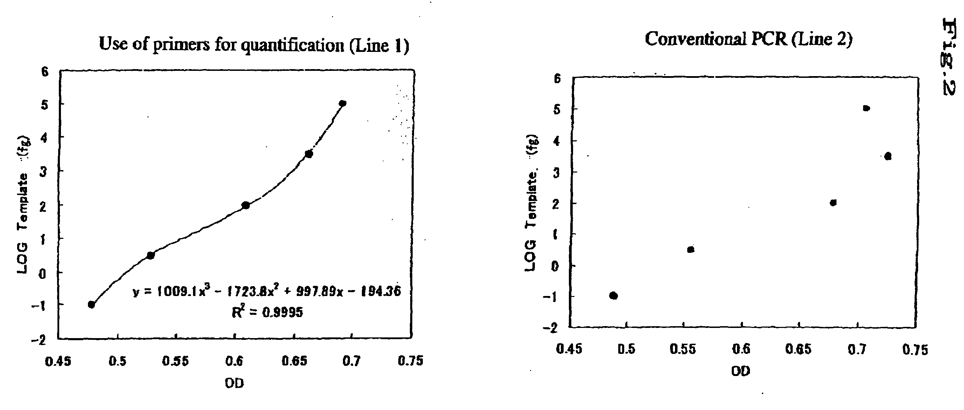

The solutions after amplification by PCR in (2) above were spotted as they were on the dry analytical element for pyrophosphoric acid quantification prepared in (3) above in amounts of 20 µl each, and the dry analytical element for pyrophosphoric acid quantification was incubated at 37°C for 5 minutes. Thereafter, the optical density of reflection (ODR) was assayed at the wavelength of 650 nm from the support side. The results are shown in Table 12. Fig. 2 shows a chart for the calibration curve of ODR relative to the exponent of the initial amount in PCR obtained from Table 12.

**Table 12: Correlation between the initial template amount in PCR and the optical density of reflection (ODR) 5 minutes later**

| Initial template amount | Optical density of reflection (ODR) | 5 minutes later |
|---|---|---|
| (fg/50 µl) | Line 1 | Line 2 |
| 0 | 0.445 | 0.442 |
| 0.1 | 0.478 | 0.488 |
| 3 x 10 | 0.529 | 0.555 |
| 1 x 10³ | 0.609 | 0.678 |
| 3 x 10⁴ | 0.662 | 0.725 |
| 1 x 10⁶ | 0.690 | 0.706 |

As is apparent from the results obtained in Example 3, no correlation is observed between the initial template amount and ODR in conventional PCR (line 2). In contrast, when a primer pair (CP0) that is complementary to the primer pair for PCR is used, there is correlation between the exponent of the initial template amount and ODR. Thus, the initial template amount can be quantified by carrying out PCR that involves the CP0 primer pair with the use of this calibration curve.

### Example 4: Quantification of an unknown initial template amount by PCR using a pyrophosphoric acid slide

### (1) Preparation of a plasmid for quantification

Solutions containing 10 pg/ µl and 10 fg/ µl of nucleic acid were prepared using the plasmid prepared in Example 3 (1), and PCR was carried out in the same manner as in Example 3. Whether or not the initial template amount was accurately quantified from the calibration curve obtained therein was examined.

### (2) Amplification by PCR

PCR was conducted for each template amount by the method described in Example 3.

### (3) Preparation of a dry analytical element for pyrophosphoric acid quantification

The dry analytical element for pyrophosphoric acid was prepared in the manner described in Example 3.

### (4) Detection using an analytical element for pyrophosphoric acid quantification

The solutions after amplification by PCR in (2) above were spotted as they were on the dry analytical element for pyrophosphoric acid quantification prepared in (3) above in amounts of 20 µL each, and the dry analytical element for pyrophosphoric acid quantification was incubated at 37°C for 5 minutes. Thereafter, the optical density of reflection (ODR) was assayed at the wavelength of 650 nm from the support side. The results are shown in Table 13. The ODR value obtained from Table 13 was rearranged using the chart for the calibration curve in Example 3 to determine the initial DNA amount. The results are shown in Table 14.

**Table 13: Correlation between the initial template amount in PCR and the optical density of reflection (ODR) 5 minutes later.**

| Initial template amount | Optical density of reflection (ODR) | 5 minutes later |
|---|---|---|
| (fg/50 µ l) | Line 1 | Line 2 |
| 0 | 0.448 | 0.445 |
| 10 | 0.552 | 0.603 |
| 1 x 10⁴ | 0.668 | 0.646 |

**Table 14: The initial template amount in PCR rearranged from Example 3**

| Initial template amount | Initial template amount calculated from the calibration curve (fg/50 µl) | |
|---|---|---|
| (fg/50 µl) | Line 1 | Line 2 |
| 0 | 0.003 | |
| 10 | 8.9 | - |
| 1x104 | 6.6 x 10³ | - |

As is apparent from the results obtained in Example 4, the obtained values are very close to the initial template amount that was first calculated. Thus, the initial template amount of PCR can be determined using a CP0 primer pair.

### Industrial Applicability

According to the method of the present invention, the target nucleic acid can be quantified in a simple and rapid manner.

## Claims

1. A method for quantifying a target nucleic acid by the polymerase chain reaction utilizing a template nucleic acid comprising a sequence of the target nucleic acid, a pair of primers for amplifying the target nucleic acid, and polymerase, wherein the polymerase chain reaction is conducted in the presence of a pair of competitive primers having sequences complementary to the sequences of the pair of amplification primers, wherein each competitive primer has, at its 5' terminus, a farther sequence of at least one or two nucleotide(s) that is not complementary to the target nucleic acid and which prevents the competitive primer from being congruous with the target nucleic acid.

2. The method according to claim 1 wherein the ratio of the competitive primer to the amplification primer is 1:100 to 1:1.

3. The method according to claim 1 wherein the 3' terminus of the competitive primer is modified in such a way that the competitive primer itself is not an initiation site for polymerase reaction

4. The method according to claim 3 wherein the 3' terminus of the competitive primer is phosphorylated, the nucleotide at the 3' terminus of the competitive primer is dideoxynucleotide, or the competitive primer has, at its 3' terminus, a farther sequence of at least one nucleotide that is not complementary to the target nucleic and which prevents the competitive primer from being congruous with the target nucleic acid.

5. The method according to claim 1 wherein the target nucleic acid in a sample is quantified by assaying the amount of the amplified product in a specified cycle in the polymerase reaction.

6. The method according to claim 4 wherein the target nucleic acid in a sample is quantified by using a calibration curve that was prepared using a known amount of nucleic acid.

7. The method according to claim 1 wherein the target nucleic acid is quantified by assaying the amplification product by electrophoresis, chromatography, or HPLC.

8. The method according to claim 1 wherein the target nucleic acid is quantified by using a reaction by-product of the polymerase reaction.

9. The method according to claim 8 wherein the reaction by-product is pyrophosphoric acid.

10. The method according to claim 9 wherein pyrophosphoric acid is detected by using a dry analytical element.

11. A pair of competitive primers to be used in the method according to claim 1, which have sequences complementary to the pair of amplification primers used in the polymerase chain reaction, wherein each competitive primer has, at its 5' terminus, a farther sequence of at least one or two nucleotide(s) that is not complementary to the target nucleic acid and which prevents the competitive primer from being congruous with the target nucleic acid.

12. The competitive primers according to claim 11 wherein the 3' terminus of the competitive primer is modified in such a way that the competitive primer itself is not an initiation site for polymerase reaction.

13. The competitive primers according to claim 12 wherein the 3' terminus of the competitive primer is phosphorylated, the nucleotide at the 3' terminus of the competitive primer is dideoxynucleotide, or the competitive primer has, at its 3' terminus, a farther sequence of at least one nucleotide that is not complementary to the target nucleic and which prevents the competitive primer from being congruous with the target nucleic acid.

## Patentansprüche

1. Verfahren zur Quantifizierung einer Target-Nucleinsäure durch die Polymerasekettenreaktion unter Verwendung einer Matrizen-Nucleinsäure, umfassend eine Sequenz der Target-Nucleinsäure, ein Paar Primer zum Amplifizieren der Target-Nucleinsäure und Polymerase, wobei die Polymerasekettenreaktion in Gegenwart eines Paares kompetitiver Primer, die Sequenzen haben, die zu den Sequenzen des Paares Amplifikationsprimer komplementär sind, wobei jeder kompetitive Primer an seinem 5'-Ende eine weitere Sequenz aus wenigstens einem oder zwei Nucleotid(en) hat, die nicht komplementär zu der Target-Nucleinsäure ist und die verhindert, dass der kompetitive Primer mit der Target-Nucleinsäure kongruent ist.

2. Verfahren gemäß Anspruch 1, wobei das Verhältnis des kompetitiven Primers zu dem Amplifikationsprimer 1:100 bis 1:1 ist.

3. Verfahren gemäß Anspruch 1, wobei das 3'-Ende des kompetitiven Primers derart modifiziert ist, dass der kompetitive Primer selbst keine Startstelle für eine Polymerasereaktion ist.

4. Verfahren gemäß Anspruch 3, wobei das 3'-Ende des kompetitiven Primers phosphoryliert ist, das Nucleotid am 3'-Ende des kompetitiven Primers Didesoxynucleotid ist oder der kompetitive Primer an seinem 3'-Ende eine weitere Sequenz aus wenigstens einem Nucleotid hat, die nicht komplementär zu der Target-Nucleinsäure ist und die verhindert, dass der kompetitive Primer mit der Target-Nucleinsäure kongruent ist.

5. Verfahren gemäß Anspruch 1, wobei die Target-Nucleinsäure in einer Probe quantifiziert wird, indem die Menge des amplifizierten Produkts in einem spezifizierten Zyklus in der Polymerasereaktion analysiert wird.

6. Verfahren gemäß Anspruch 4, wobei die Target-Nucleinsäure in einer Probe quantifiziert wird, indem eine Eichkurve verwendet wird, die unter Verwendung einer bekannten Menge an Nucleinsäure erstellt wurden.

7. Verfahren gemäß Anspruch 1, wobei die Target-Nucleinsäure quantifiziert wird, indem das Amplifikationsprodukt durch Elektrophorese, Chromatographie oder HPLC analysiert wird.

8. Verfahren gemäß Anspruch 1, wobei die Target-Nucleinsäure durch Verwendung eines Reaktionsnebenprodukts der Polymerasereaktion quantifiziert wird.

9. Verfahren gemäß Anspruch 8, wobei das Reaktionsnebenprodukt Pyrophosphorsäure ist.

10. Verfahren gemäß Anspruch 9, wobei Pyrophosphorsäure durch Verwendung eines Trockenanalyseelements detektiert wird.

11. Paar kompetitiver Primer, die in dem Verfahren gemäß Anspruch 1 zu verwenden sind, welche Sequenzen haben, die komplementär zu dem Paar Amplifikationsprimer, die in der Polymerasekettenreaktion verwendet werden, sind, wobei jeder kompetitive Primer an seinem 5'-Ende eine weitere Sequenz aus wenigstens einem oder zwei Nucleotid(en) hat, die nicht komplementär mit der Target-Nucleinsäure ist und die verhindert, dass der kompetitive Primer mit der Target-Nucleinsäure kongruent ist.

12. Kompetitive Primer gemäß Anspruch 11, wobei das 3'-Ende des kompetitiven Primers derart modifiziert ist, dass der kompetitive Primer selbst keine Startstelle für eine Polymerasereaktion ist.

13. Kompetitive Primer gemäß Anspruch 12, wobei das 3'-Ende des kompetitiven Primers phosphoryliert ist, das Nucleotid am 3'-Ende des kompetitiven Primers Didesoxynucleotid ist oder der kompetitive Primer an seinem 3'-Ende eine weitere Sequenz aus wenigstens einem Nucleotid hat, die nicht komplementär zu der Nucleinsäure ist und die verhindert, dass der kompetitive Primer mit der Target-Nucleinsäure kongruent ist.

## Revendications

1. Méthode pour quantifier un acide nucléique de cible par réaction en chaîne de polymérase en utilisant un acide nucléique matrice comprenant une séquence de l'acide nucléique de cible, un couple d'amorces pour amplifier l'acide nucléique de cible et de la polymérase, dans laquelle la réaction en chaîne de polymérase est réalisée en présence d'un couple d'amorces compétitives ayant des séquences complémentaires aux séquences du couple d'amorces d'amplification, dans laquelle chacune amorce compétitive possède, à l'extrémité 5', une séquence additionnelle composée d'au moins un ou deux nucléotide(s) qui n'est pas complémentaire à l'acide nucléique de cible et qui empêche l'amorce compétitive d'être congruente à l'acide nucléique de cible.

2. Méthode selon la revendication 1, dans laquelle le rapport de l'amorce compétitive à l'amorce d'amplification est de 1:100 à 1:1.

3. Méthode selon la revendication 1, dans laquelle l'extrémité 3' de l'amorce compétitive est modifiée de telle manière que l'amorce compétitive elle-même n'est pas l'endroit d'initiation pour la réaction de polymérase.

4. Méthode selon la revendication 3, dans laquelle l'extrémité 3' de l'amorce compétitive est phosphorylée, le nucléotide à l'extrémité 3' de l'amorce compétitive est un didésoxynucléotide ou l'amorce compétitive possède, à son extrémité 3', une séquence additionnelle composée d'au moins un nucléotide qui n'est pas complémentaire à l'acide nucléique de cible et qui empêche l'amorce compétitive d'être congruente à la cible nucléique de cible.

5. Méthode selon la revendication 1, dans laquelle l'acide nucléique de cible dans un échantillon est quantifié en déterminant la quantité du produit amplifié dans un cycle spécifié dans la réaction de polymérase.

6. Méthode selon la revendication 4, dans laquelle l'acide nucléique de cible dans un échantillon est quantifié en utilisant une courbe d'étalonnage qui était préparée en utilisant une quantité connue d'acide nucléique.

7. Méthode selon la revendication 1, dans laquelle l'acide nucléique de cible est quantifié en analysant le produit d'amplification par électrophorèse, chromatographie ou CLHP.

8. Méthode selon la revendication 1, dans laquelle l'acide nucléique de cible est quantifié en utilisant un sous-produit de la réaction de polymérase.

9. Méthode selon la revendication 8, dans laquelle le sous-produit est l'acide pyrophosphorique.

10. Méthode selon la revendication 9, dans laquelle l'acide pyrophosphorique est détecté en utilisant un élément analytique sec.

11. Couple d'amorces compétitives pour l'utilisation dans la méthode selon la revendication 1, ayant des séquences complémentaires aux couples d'amorces d'amplification utilisés dans la réaction en chaîne de polymérase, dans laquelle chacune amorce compétitive possède, à son extrémité 5', une séquence additionnelle composée d'au moins un ou deux nucléotides(s) qui n'est pas complémentaire à l'acide nucléique de cible et qui empêche l'amorce compétitive d'être congruente à l'acide nucléique de cible.

12. Amorces compétitives selon la revendication 11, l'extrémité 3' de l'amorce compétitive étant modifiée de telle manière que l'amorce compétitive elle-même n'est pas un endroit d'initiation pour la réaction de polymérase.

13. Amorces compétitives selon la revendication 12, dans lesquelles l'extrémité 3' de l'amorce compétitive est phosphorylée, le nucléotide à l'extrémité 3' de l'amorce compétitive est un didésoxynucléotide ou l'amorce compétitive possède, à son extrémité 3', une séquence additionnelle d'au moins un nucléotide, qui n'est pas complémentaire à l'acide nucléique et qui empêche l'amorce compétitive d'être congruente à l'acide nucléique de cible.


### SEQUENCE LISTING

<110> FUJI PHOTO FILM CO., LTD.
<120> A METHOD FOR QUANTIFYING A TARGET NUCLEIC ACID
<130> 13825EP
<140>
   <141>
<150> JP 360914/2002
   <151> 2002-12-12
<160> 8
<170> PatentIn Ver. 2.1
<210> 1
   <211> 20
   <212> DNA
   <213> Artificial Sequence
<220>
   <223> Description of Artificial Sequence:Primer
<400> 1
   gggcatgggt cagaaggatt 20
<210> 2
   <211> 20
   <212> DNA
   <213> Artificial Sequence
<220>
   <223> Description of Artificial Sequence:Primer
<400> 2
   ccgtggtggt gaagctgtag 20
<210> 3
   <211> 22
   <212> DNA
   <213> Artificial Sequence
<220>
   <223> Description of Artificial Sequence:Primer
<400> 3
   ttaatccttc tgacccatgc cc 22
<210> 4
   <211> 22
   <212> DNA
   <213> Artificial Sequence
<220>
   <223> Description of Artificial Sequence:Primer
<400> 4
   ttctacagct tcaccaccac gg 22
<210> 5
   <211> 20
   <212> DNA
   <213> Artificial Sequence
<220>
   <223> Description of Artificial Sequence:Primer
<400> 5
   gggcatgggt cagaaggatt 20
<210> 6
   <211> 20
   <212> DNA
   <213> Artificial Sequence
<220>
   <223> Description of Artificial Sequence:Primer
<400> 6
   ccgtggtggt gaagctgtag 20
<210> 7
   <211> 20
   <212> DNA
   <213> Artificial Sequence
<220>
   <223> Description of Artificial Sequence:Primer
<400> 7
   aatccttctg acccatgccc 20
<210> 8
   <211> 20
   <212> DNA
   <213> Artificial Sequence
<220>
   <223> Description of Artificial Sequence:Primer
<400> 8
   ctacagcttc accaccacgg 20